Europäisches Patentamt

European Patent Office

Office européen des brevets

(11) Publication number: **0 269 928**

**A2**

(12) **EUROPEAN PATENT APPLICATION**

(21) Application number: 87116752.4

(51) Int. Cl.4: **A61K 39/02** , A61K 39/116

(22) Date of filing: 13.11.87

(30) Priority: 21.11.86 IT 4867786

(43) Date of publication of application:
08.06.88 Bulletin 88/23

(84) Designated Contracting States:
AT CH DE ES FR GB GR IT LI LU NL SE

(71) Applicant: ISTITUTO SIEROTERAPICO
MILANESE "S. BELFANTI"
Via Darwin, 22
I-20143 Milano(IT)

(72) Inventor: Monti, Giuseppe
Via Darwin, 22
I-20143 Milano(IT)

(74) Representative: Minoja, Fabrizio
Studio Consulenza Brevettuale Via Rossini,
8
I-20122 Milano(IT)

(54) Bacterial antigenic lysate, a process for the preparation thereof and pharmaceutical compositions containing it.

(57) A bacterial antigenic lysate prepared by subjecting to lysis selected bacterial strains, particularly Staphylococcus aureus, Streptococcus pyogenes, Streptococcus viridans, Diplococcus pneumoniae, Klebsiella pneumoniae, Klebsiella ozaenae, Haemophilus influenzae type B, Neisseria catarrhalis, is described.

The obtained lysates are useful as vaccines to be administered by the oral route for the treatment of infective diseases of the respiratory tract, particularly to prevent relapses and transformations into chronic conditions.

EP 0 269 928 A2

## BACTERIAL ANTIGENIC LYSATE, A PROCESS FOR THE PREPARATION THEREOF AND PHARMACEUTICAL COMPOSITIONS CONTAINING IT

The present invention relates to a bacterial antigenic lysate useful as a vaccine for the treatment and prophylaxis of infective diseases of the respiratory tract.

Vaccines consisting of bacterial lysates are known and already used to prevent various infective diseases.

A main problem of such products resides in the selection of the bacterial species, whose immunologic features are of primary importance to determine the preventive and/or therapeutic effectiveness of the vaccines obtained therefrom.

Now it has been found an improved and more effective vaccine in comparison with the ones hitherto known, said vaccine consisting in lysates from the following bacterial strains: Staphylococcus aureus, Streptococcus pyogenes, Streptococcus viridans, Diplococcus pneumoniae, Klebsiella pneumoniae, Klebsiella ozaenae, Haemophilus influenzae type B, Neisseria catarrhalis.

The vaccine object of the present invention turned out to be particularly active in the prevention and as an adjuvant in the treatment of acute and chronic bronchitis, anginas, tonsillitis, pharyngitis and laryngitis, rhinitis, sinusitis, otitis, infections resisting to conventional antibiotics, bacterial complications of viral infections of the respiratory tract, particularly in the elderly.

Bacterial lysates may be advantageously administe red by the oral route, in form of solid or liquid pharmaceutical compositions. Capsules and tables prepared using conventional excipients and techniques, such as those described in "Remington's Pharmaceutical Sciences Handbook", Hack Publ., New York, USA, are particularly preferred.

Each lysate is present in the vaccine according to the invention in amounts from 1 to 10 thousand millions bacteria. The preferred bacterial count is of 6 thousand millions per single strain.

In the treatment of acute conditions one administration a day for at least 10 days is advisable, while in the follow up treatment it is suitable to repeat a 10 days cycle a month, for 3 months.

For the preparation of the lysates and vaccine according to the invention, the bacterial strains listed in the following Table are used.

| Bacterium | Code |
|---|---|
| Staphylococcus aureus | ISM 68/211 |
| Streptococcus pyogenes | ISM 80/21 |
| Streptococcus viridans | ISM 70/1 |
| Diplococcus pneumoniae | ISM 73/27;73/28;70/69; 73/31;82/43;82/44; |
| Klebsiella pneumoniae | ISM 80/5 |
| Klebsiella ozaenae | ISM 84/87 |
| Haemophilus influenzae type B | ISM 82/49 |
| Neisseria catarrhalis | ISM 70/23 |

The above reported strains, after reconstitution from the lyophil in Todd-Hewitt medium, were incubated at 30°C for 10 hours, after which the broth culture was incu bated in the same medium at 37°C for 24-30 hours After successive scale passages, the obtained bacterial suspension was concentrated by flow-through centrifugation.

Lysis was then carried out by incubation in thermostat at 37°C for 7 days, adjusting pH of the suspension between 11 and 12.

The obtained lysate suspensions were then added with .50% w/v citric acid to adjust pH to 7 and then were filtered in sterile conditions. Finally, lyophilization was effected on suitable substrates, preferably glycine.

The following non-limiting examples describe in more detail the procedure to obtain a lysate of Streptococcus pyogenes; of course, lysates of the other bacteria which constitute the vaccine are obtained analogously, in case replacing Todd-Hewitt medium with the casein and soy hydrolysate medium.

## EXAMPLE 1

### 1) Lysate of Streptococcus pyogenes

a) Strain: the ISM 80/21 strain of Streptococcus pyogenes kept in the lyophilized state, was used.

b) Pre-culture: the strain was re-constituted from the lyophil in liquid Todd-Hewitt medium and incubated at 37°C for 10 hours, after which the broth culture was poured into a 1 liter flask containing 200 ml of the same medium. The flask was placed on a rotating surface (120 r.p.m.) at the temperature of +37°C for 24-30 hours.

c) Fermentation: at the end of incubation, the contents of the flask were seeded in a 141 fermenter (manufactured by CHEMAP) containing 10 liters of the same medium. Broth culture was incubated for 24 hours at +37°C. under mild electromechanical stirring and aeration by injection of sterile air. After having obtained a positive outcome from the purity check by bacterioscopic examination, the contents of the fermenter were transferred, through sterile lines, into a 250 I fermenter (CHEMAP) containing 200 I of liquid Todd-Hewitt medium. Broth culture was subjected to the same conditions described for the pilot-fermenter. 24 hours after the culture was checked for purity by bacterioscopic examination.

d) Preparation of the concentrated bacterial suspension.
After a positive checking, the bacterial suspension was poured into a 400 I reactor and subjected to a concentration treatment by means of a flow-through Toniatti centrifuge. The deposit was successively re-suspended into 5 I of sterile distilled water. The concentrated bacterial suspension was thus obtained, which was successively checked.

e) Preparation of the bacterial lysate suspension.
After positive checking, the suspension was adjusted to pH betweeen 11 and 12 by addition of a 10 N sodium hydroxide solution. After then, the suspension was subjected to lysis in thermostat at +37°C, with stirring twice a day. After 7 days incubation, pH was checked again, re-adjusted to 11-12 if case, and subsequently a sample was withdrawn to check the bacterial count in order to evaluate the number of unlysated bacteria.

### 2) Lyophilization of bacterial lysates

The lysate suspensions of each strain were admixed in a 300 I stainless steel reactor. pH of the mixture was adjusted to 7 by addition of a 50% w/v citric acid solution, then, through a sterile line, the mixture was filtered by a 0.22 mcm sterilizing membrane and distributed into 35 big glass flasks of 10 I capacity (about 7.8 I per flask).

A sample was withdrawn form each flask to be checked for sterility (see par. 4.1.3).

After a positive checking, 600 ml of a 20% w/v glycine solution was added to each flask.

After appropriate stirring, the mixture was partitioned into suitable trays and subsequently lyophilized in Teknolabo apparatus. The thus obtained powder was weighed and stored in polythene bags under vacuum at 6°C.

## EXAMPLE 2

Pharmaceutical formulation in tablets.

Qualitative and quantitative composition: One tablet contains:

Lyophilized bacterial lysate 50 mg, 7 mg of which corresponded to:
Staphylococcus aureus        6 thousand millions
Streptococcus pyogenes      6 thousand millions
Streptococcus viridans      6 thousand millions

Diplococcus pneumoniae      6 thousand millions
Klebsiella pneumoniae      6 thousand millions
Klebsiella ozaenae        6 thousand millions
Haemophilus influenzae type B      6 thousand millions
Neisseria catarrhalis      6 thousand millions
Lyophilization substrate: glycine      43 mg
Excipients:
Silicium dioxide                10 mg
Microcrystalline cellulose        150 mg
Calcium phosphate dibasic          35 mg
Magnesium stearate              3 mg
Ammonium glycyrrhizinate          1,2 mg
Powdered mint flavor            0.8 mg

**Claims**

1. A vaccine comprising antigenic bacterial lysate obtained from Staphylococcus aureus, Streptococcus pyogenes, Streptococcus viridans, Diplococcus pneumoniae, Klebsiella pneumoniae, Klebsiella ozaenae, Haemophilus influenzae type B, Neisseria catarrhalis.

2. A vaccine as claimed in claim 1, in form of capsules or tablets for oral administration.

3. A vaccine as claimed in claim 1 or 2, wherein each lysate from each single strain is present in amounts corresponding to a bacterial count from 1 to 10 thousand millions.

4. A process for the preparation of bacterial lysates of Staphylococcus aureus, Streptococcus pyogenes, Streptococcus viridans, Diplococcus pneumoniae, Klebsiella pneumoniae, Klebsiella ozaenae, Haemophilus influenzae type B, Neisseria catarrhalis, which process comprises the following steps:

a) culture of bacterial strains in Todd-Hewitt medium;

b) concentration of the obtained bacterial suspension by means of flow-through centrifugation;

c) lysis at pH 11-12 during 7 days at 37°C;

d) sterile filtration at pH 7 and lyophilization.